Europäisches Patentamt

**European Patent Office**

**Office européen des brevets**

(19)

(11) Numéro de publication : **0 004 216
B1**

(12) **FASCICULE DE BREVET EUROPÉEN**

(45) Date de publication du fascicule du brevet :
21.10.81

(21) Numéro de dépôt : **79400103.2**

(22) Date de dépôt : **20.02.79**

(51) Int. Cl.³ : **E 04 B  1/62, E 04 D  5/08//
A01G9/14**

(54) **Revêtement pour isoler thermiquement et protéger une construction ou une structure rigides contre les intempéries et utilisation d'un tel revêtement pour les maisons d'habitation.**

(30) Priorité : **07.03.78 FR 7806426**

(43) Date de publication de la demande :
**19.09.79 (Bulletin 79/19)**

(45) Mention de la délivrance du brevet :
**21.10.81 Bulletin 81/42**

(84) Etats contractants désignés :
**BE CH DE FR GB IT LU NL SE**

(56) Documents cités :
**CH - A - 333 815
FR - A - 533 131
FR - A - 1 536 966
FR - A - 2 139 053
FR - A - 2 292 078
GB - A - 916 016
US - A - 3 368 473
US - A - 3 806 391**

(73) Titulaire : **ETABLISSEMENTS D'ARDOREL Société
Anonyme française
Payrin
F-81200 Mazamet (FR)**

(72) Inventeur : **Cros, Jacques
Cambon
F-81210 Roquecourbe (FR)**

(74) Mandataire : **Bouju, André
Cabinet Bouju 38 avenue de la Grande Armée
F-75017 Paris (FR)**

EP 0 004 216 B1

Imprimerie Jouve, 17, rue du Louvre, 75001 Paris, France

## Revêtement pour isoler thermiquement et protéger une construction ou une structure rigides contre les intempéries et utilisation d'un tel revêtement pour les maisons d'habitation

La présente invention concerne un revêtement souple confectionné à l'aide de fibres minérales pour isoler thermiquement et protéger une construction ou une structure rigides contre les intempéries telles que vent et pluie.

L'invention vise également l'utilisation d'un revêtement pour assurer l'isolation et la protection précitées.

Par construction, on entendra, notamment, les maisons et immeubles d'habitation ou à usage professionnel, les bâtiments et hangars industriels ou agricoles, ainsi que les serres, vérandas et abris analogues, y compris leur charpente.

On connaît de nombreux procédés pour isoler thermiquement les constructions. Dans la plupart des procédés, on dispose, sur la face intérieure des murs ou du toit d'une construction, des plaques de matière isolante telle que le polystyrène expansé ou de la laine de verre. Dans certains procédés, on dispose de telles plaques ou de la laine de verre à l'intérieur du mur, par exemple, entre deux rangées de parpaings ou de briques. La face extérieure des murs est protégée le plus souvent par un enduit de mortier complété par une mince couche de finition d'enduit synthétique.

On connaît, d'autre part, selon le FR-A-2 139 053, un procédé consistant à revêtir l'extérieur des murs d'une maison par un revêtement souple imperméable en fibres, par exemple de verre, liées ensemble par une résine synthétique et présentant l'apparence extérieure d'une fourrure. Un tel revêtement apporte une certaine isolation thermique et phonique. Cependant, ce revêtement est onéreux, il retient l'humidité en favorisant les moisissures et se dégrade rapidement sous l'effet conjoint de l'humidité et du rayonnement solaire.

Dans le cas des serres, la protection contre le vent et la pluie, de même que l'isolation thermique, est assurée par des feuilles de verre ou de matière plastique appliquées et fixées directement sur l'armature de la serre.

Les feuilles de verre engendrent, sous l'action du rayonnement solaire, un réchauffement du volume intérieur, selon l'effet « de serre » bien connu. Toutefois, ces feuilles de verre présentent l'inconvénient d'être fragiles et peu pratiques à mettre en œuvre.

Les feuilles de matière plastique, comme par exemple selon le FR-A-2 292 078, telles que le polyéthylène ou le chlorure de polyvinyle sont beaucoup plus commodes à mettre en œuvre, en raison de leur souplesse, mais elles procurent un effet de serre moins important que le verre, et surtout elles vieillissent assez rapidement sous l'action du rayonnement solaire.

Les feuilles de verre et de matière plastique présentent, de plus, l'inconvénient d'être imperméables à l'air, de sorte qu'il est nécessaire de pratiquer dans les serres des ouvertures pour aérer l'intérieur de ces dernières.

Le but de l'invention est de fournir un revêtement pour isoler thermiquement et protéger efficacement une construction ou structure rigides contre les intempéries, notamment contre la pluie et le vent, tout en permettant une ventilation du volume protégé.

Suivant l'invention, ce revêtement est caractérisé en ce qu'il est constitué par un tricot en fils de fibres minérales dont les mailles sont suffisamment lâches pour permettre le passage de l'air, mais suffisamment serrées pour diviser ou calibrer les gouttes d'eau de pluie.

Un tel revêtement résiste d'une manière exceptionnelle au vieillissement par l'eau et les rayons ultraviolets. Ce revêtement présente, d'autre part, une excellente résistance mécanique due au tricotage des fils en fibres minérales. En effet, le tricotage forme des boucles, sans angle droit, favorables à la tenue mécanique et à la souplesse du revêtement. De plus, les mailles d'un tel tricot définissent des pores perméables à l'air.

Selon une version préférée du revêtement conforme à l'invention, le tricot est en fils de fibres de verre.

La mise en œuvre d'un tel tricot en fils de fibres de verre est très commode, compte tenu de sa résistance et de sa déformabilité. De plus, ce tricot en fils de fibres de verre présente une tenue remarquable au vieillissement, à l'égard des agents atmosphériques et du rayonnement solaire. En outre, l'expérience a montré ce résultat surprenant, qu'un tricot du genre considéré assurait l'isolation thermique de l'extérieur de la construction, tout en constituant une barrière vis-à-vis de la pluie et du vent.

L'air traverse le tricot de fils de fibres de verre par les pores de ce dernier, à un débit modéré, mais suffisant pour éviter la condensation d'humidité. Cette condensation d'humidité est également évitée par l'effet de serre qui est créé dans l'espace précité par interaction entre le rayonnement solaire et le tricot en fils de fibres de verre. Ce tricot en fils de fibres de verre protège également les murs et le toit des maisons de l'action érosive du vent chargé de poussières abrasives. Le verre a, en effet, une excellente résistance à l'abrasion.

De plus, un tel tricot de fils de fibres de verre présente les avantages d'être souple et facile à manipuler comme les feuilles de matière plastique utilisées jusqu'à présent, sans toutefois présenter la faible résistance au vieillissement de ces derniers sous l'action de l'ultraviolet solaire.

De préférence, le revêtement conforme à l'invention est constitué par un tricot en fibres de verre dont les mailles sont suffisamment serrées pour éviter le passage de l'eau de pluie tout en permettant le passage de l'air.

Un tel tricot peut être obtenu aisément au moyen d'un métier à tricoter du type RACHEL, tel que décrit par exemple dans le livre intitulé « Tricot et Métier Rachel » par M. Meuris, édition La Maille (Paris).

L'invention vise également l'utilisation du revêtement précité pour l'isolation thermique et la protection contre le vent des maisons d'habitation. Selon cette utilisation, on recouvre le toit et les murs de ces maisons au moyen dudit revêtement, en laissant un espace vide entre le revêtement, le toit et les murs.

D'autres particularités et avantages de l'invention apparaîtront encore dans la description ci-après.

Aux dessins annexés, donnés à titre d'exemples non limitatifs :

— la figure 1 est une vue en perspective avec arrachements d'une maison recouverte par un revêtement conforme à l'invention ;

— la figure 2 est une vue en coupe transversale partielle à plus grande échelle du toit et d'un mur de la maison précédente ;

— la figure 3 est une vue partielle, à échelle agrandie, montrant un tricot en fils de fibres de verre fixé à un liteau ;

— la figure 4 est une vue en coupe transversale, à grande échelle, d'un tricot en fils de fibres de verre collé sur un liteau ;

— la figure 5 est une vue en coupe transversale d'un revêtement à plusieurs couches de tricot en fils de fibres de verre ;

— la figure 6 est une vue en perspective d'une serre recouverte par un revêtement conforme à l'invention.

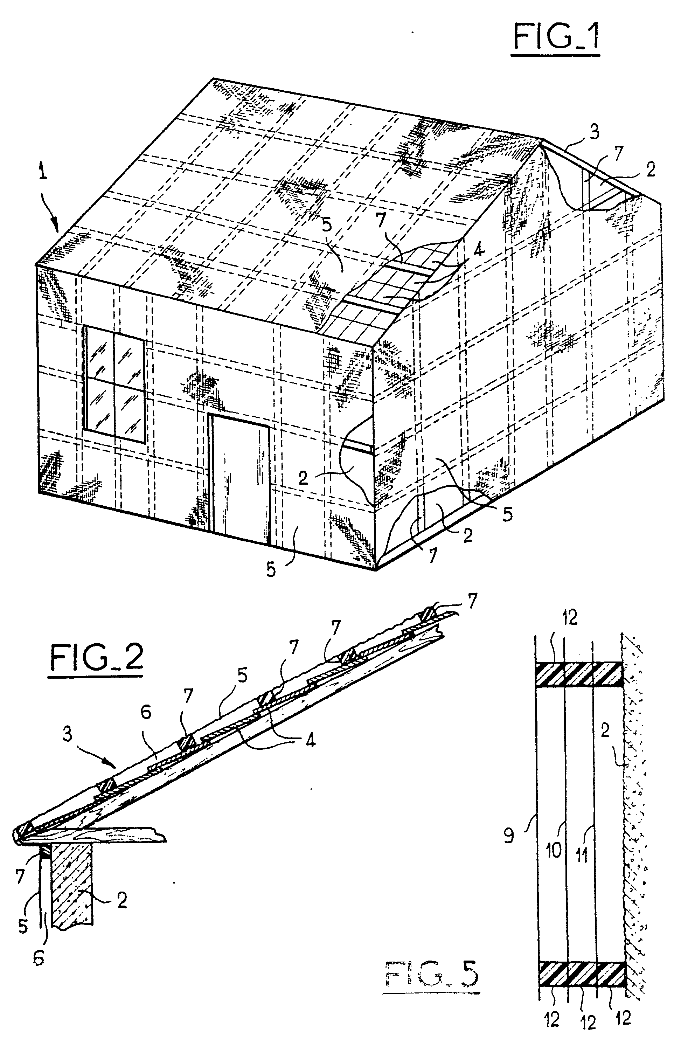

En référence à la figure 1, on a représenté une maison 1 comprenant des murs latéraux 2 et un toit 3 recouvert de tuiles 4.

Pour améliorer l'isolation thermique et protéger les murs 2 et le toit 3 contre les effets de la pluie et du vent, on a recouvert l'extérieur de la maison 1, c'est-à-dire les murs 2 et le toit 3 par un revêtement 5 constitué par un tricot en fils de fibres de verre.

Les fils de ce tricot sont obtenus en tordant, dans le même sens, un certain nombre de filaments composés de fibres de verre. Plusieurs de ces fils peuvent être retordus ensemble, notamment par torsion inverse pour obtenir un fil retors très solide.

Compte tenu de la résistance mécanique élevée et de la souplesse du tricot en fils de fibres de verre, cette opération peut être réalisée en déroulant sur le toit 3 et sur les murs 2 des panneaux en tricot en fils de fibres de verre prédécoupés aux dimensions appropriées.

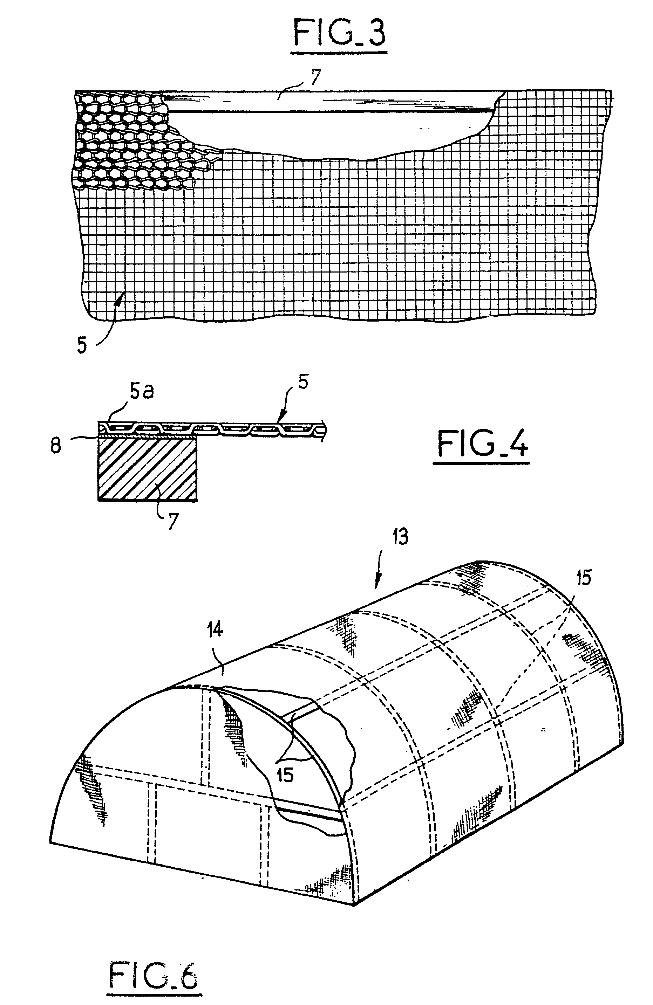

Dans le cas particulier d'une maison, il est préférable de laisser un espace vide 6 entre le tricot en fils de fibres de verre 5, le toit 3 et les murs 2 (figure 2). Pour réaliser cet espace 6, on peut fixer sur le toit 3 et les murs 2, des liteaux 7 régulièrement espacés. Le tricot en fils de fibres de verre peut être fixé sur ces liteaux 7 (figure 4) par exemple par clouage ou agrafage. Il est préférable, toutefois, de fixer le tricot en fils de fibres de verre 5 sur les liteaux 7 au moyen d'une couche de colle mécaniquement résistante 8, comme indiqué sur la figure 4. Cette couche de colle 8 empêche l'ouverture des mailles du tricot 5 à son bord 5a adjacent au liteau 7.

Il est possible, d'autre part, de fixer les panneaux prédécoupés de tricot 5 sur les liteaux 7, avant de fixer ces derniers sur les murs 2 et le toit 3 de la maison 1.

Dans ce cas, il est avantageux que les liteaux 7 soient constitués par des profilés souples, par exemple en chlorure de polyvinyle, ce qui permet d'appliquer facilement le revêtement 5 sur des surfaces courbes. De tels profilés souples peuvent être moulés directement sur les deux bords longitudinaux du ruban de tricot en fils de fibres de verre à sa sortie du métier à tricoter.

L'épaisseur de l'espace vide 6 laissé entre les murs 2, le toit 3 et le tricot 5 est, de préférence, suffisante pour éviter tout contact entre le tricot 5, le mur 2 et le toit 3, compte tenu des pressions susceptibles de s'exercer sur ce tricot sous l'effet, par exemple, du vent, ou de la pluie. A cet égard, des liteaux 7, d'épaisseur égale à 5 centimètres et espacés d'environ un mètre, peuvent convenir dans la plupart des cas.

Le tricot en fils de fibres de verre 5 peut être fabriqué au moyen d'un métier à tricoter du type RACHEL, à partir de fils en fibres de verre d'épaisseur comprise entre 0,5 et 2 millimètres environ. L'armure du tricot 5 peut être, par exemple, du type jersey. La maille jersey est la plus simple et c'est celle qui fournit le meilleur rapport solidité/poids. On obtient ainsi un tricot 5 d'épaisseur comprise entre 1 et 4 millimètres, présentant une très grande résistance à la traction.

Les mailles du tricot sont, généralement, suffisamment serrées pour diviser ou calibrer les gouttes d'eau de pluie, tout en étant suffisamment lâches pour laisser passer l'eau. L'expérience a montré que pour revêtir les murs, il y avait avantage que les dimensions moyennes des mailles du tricot 5 soient comprises entre 2 et 6 fois le diamètre du fil.

Dans certains cas, toutefois, comme par exemple pour revêtir les toits, il y a avantage à choisir des mailles relativement serrées (dimensions inférieures à 2 fois le diamètre du fil). Dans ce cas, l'eau de pluie ne traverse pas le tricot 5, mais ce dernier reste perméable à l'air, de sorte que l'on évite les moisissures sous les toits.

Les avantages et les effets techniques du tricot en fils de fibres de verre 5, appliqué sur les murs 2 et le toit 3 de la maison 1, sont les suivants :

Compte tenu de la souplesse du tricot 5 (comparable à celle d'un tricot en fils textiles classiques), et par suite, de son excellente maniabilité, la protection extérieure de la maison 1 peut être réalisée en très peu de temps, sans apporter aucune modification à l'extérieur de la maison 1. La souplesse du tricot s'explique par les boucles de ce tricot, et l'absence d'angle droit.

Le tricot en fils de fibres de verre 5 est transparent au rayonnement solaire et engendre un effet de serre comparable au verre, sans présenter l'inconvénient majeur de ce dernier, c'est-à-dire sa fragilité. Cet effet de serre, et la couche d'air 6 comprise entre le tricot 5, les murs 2 et le toit 3, sont bénéfiques pour l'isolation thermique.

D'autre part, le tricot en fils de fibres de verre présente un coefficient de dilatation thermique sensiblement nul, entre − 30 °C et + 40 °C, de sorte qu'il reste toujours parfaitement tendu.

Le tricot en fils de fibres de verre 5 constitue une barrière très efficace contre le vent dont l'action érosive et l'effet d'accélération des échanges thermiques sont ainsi supprimés.

Les pores du tricot 5 permettent une aération continuelle de l'espace 6 compris entre le tricot 5, les murs 2 et le toit 3. On évite ainsi les condensations d'humidité susceptibles d'entraîner des moisissures, et par suite, la dégradation des murs 2, des tuiles 4 et de la charpente de la maison 1.

Les pores du tricot 5 de faible dimension évitent le passage des gouttelettes d'eau de pluie.

Par temps humide et très froid, il se forme, à la surface du tricot 5, une pellicule de givre ou de glace hermétique, favorable à l'isolation thermique, compte tenu de la chaleur latente de solidification de la glace.

Dans les régions particulièrement froides, l'isolation thermique peut être améliorée en appliquant, à l'extérieur de la maison, notamment sur les parois les plus exposées de celle-ci, plusieurs couches 9, 10, 11, de tricot en fils de fibres de verre, de préférence séparées par des liteaux 12, comme indiqué sur la figure 5.

Complémentairement, le revêtement conforme à l'invention, dans la mesure où il protège du vent, protège également très efficacement les constructions contre les incendies survenant à l'extérieur de ces dernières, en particulier lorsque celles-ci sont réalisées en partie ou en totalité en matériaux inflammables.

D'autre part, le revêtement conforme à l'invention du fait de sa structure à mailles constitue un excellent support pour l'accrochage des plantes grimpantes telles que lierre, chèvrefeuille, vigne, qui apportent une isolation thermique complémentaire, sans aucun risque de dégradation des murs.

L'invention s'applique également à la protection des serres.

Dans la réalisation de la figure 6, le tricot en fils de fibres de verre 14 est appliqué directement sur l'armature métallique 15 qui forme la structure de la serre 13.

Compte tenu de sa solidité, de sa souplesse et de son excellente résistance au vieillissement, ce tricot en fils de fibres de verre 14 remplace avantageusement les feuilles de verre et de matière plastique utilisées jusqu'à présent.

De plus, les pores calibrés du tricot en fils de fibres de verre 14, tout en évitant le passage des gouttes d'eau de pluie, permettent une circulation d'air à un flux modéré mais suffisant, pour assurer une aération régulière et contrôlée de l'intérieur de la serre 13.

Bien entendu, l'invention n'est pas limitée aux exemples que l'on vient de décrire, et on peut apporter à ceux-ci de nombreuses modifications sans sortir du cadre de l'invention.

Ainsi, le revêtement conforme à l'invention peut encore s'appliquer à la protection extérieure des hangars industriels et agricoles en bois ou en métal.

Le tricot en fils de fibres de verre 5, 9, 10, 11, 14, peut encore être revêtu par une résine résistant bien au vieillissement à l'égard des effets conjoints de l'eau et du rayonnement ultraviolet, tel qu'une résine acrylique, après sa mise en place sur la construction, en évitant toutefois d'obturer complètement les pores compris entre les fils de fibres de verre du tricot pour permettre le passage de l'air.

Cette enduction de résine acrylique permet d'améliorer la transparence du tricot en fils de fibres de verre en rendant ainsi celui-ci pratiquement invisible, comme le verre. Cette enduction peut être favorable dans les applications dans lesquelles on ne désire pas modifier l'aspect esthétique d'origine de la construction.

Bien entendu, il est possible de peindre le tricot en fils de fibres de verre dans les couleurs correspondant à celles des murs et des toits des maisons sans obstruer les pores du tricot. Dans ce cas, on réduit toutefois la transparence du tricot en fils de fibres de verre, et de ce fait, l'effet de serre. On peut également appliquer le tricot en plusieurs couches superposées. On peut également prévoir que le tricot présente, en certains endroits, des mailles plus lâches pour permettre une meilleure aération ou transparence du revêtement conforme à l'invention.

Les fils en fibres de verre d'un tel tricot peuvent être remplacés par d'autres fibres minérales pouvant être tricotées, telles que la laine de roche, les fibres céramiques et éventuellement les fibres de carbone. Toutes ces fibres confèrent au revêtement-tricoté conforme à l'invention, une structure aérée stable au cours du temps à l'égard des intempéries du rayonnement solaire et des variations de température.

## Revendications

1. Revêtement souple confectionné à l'aide de fibres minérales pour isoler thermiquement et protéger une construction ou une structure rigide contre les intempéries telles que vent et pluie, ce revêtement recouvrant l'extérieur de la construction (1) ou de la structure (15), caractérisé en ce qu'il est constitué par un tricot (5) en fils de fibres minérales, les mailles de ce tricot (5) étant suffisamment lâches pour permettre le passage de l'air, tout en étant suffisamment serrées pour diviser ou calibrer les gouttes d'eau de pluie.

2. Revêtement conforme à la revendication 1, caractérisé en ce que le tricot (5) est en fils de fibres de verre.

3. Revêtement conforme à l'une quelconque des revendications 1 ou 2, caractérisé en ce que le tricot (5) est enduit par une résine présentant une excellente résistance au vieillissement par l'eau et le rayonnement ultra-violet, cette résine n'obturant pas complètement les interstices compris entre les fils de fibres minérales.

4. Revêtement conforme à l'une quelconque des revendications 1 à 3, caractérisé en ce que les mailles du tricot (5) sont suffisamment serrées pour éviter le passage de l'eau, tout en permettant le passage de l'air.

5. Revêtement conforme à la revendication 4, caractérisé en ce qu'il est constitué par plusieurs couches (9, 10, 11) de tricot (5).

6. Revêtement conforme à la revendication 5, caractérisé en ce qu'il est constitué par plusieurs couches (9, 10, 11) de tricot (5) séparées par des liteaux (12).

7. Utilisation du revêtement conforme à l'une quelconque des revendications 1 à 6, pour l'isolation thermique et la protection contre le vent des maisons d'habitation (1), caractérisée en ce qu'on recouvre le toit (3) et les murs (2) de ces maisons (1) au moyen dudit revêtement constitué par le tricot (5), en laissant un espace vide (6) entre le tricot (5), le toit (3) et les murs (2).

## Claims

1. A flexible covering made of mineral fibres for the heat insulation and the protection of a construction or a rigid structure against intemperateness such as wind and rain, this covering covering the exterior of the construction (1) or the structure (15), characterized in that it is constituted by a *knitted* fabric (5) of threads of mineral fibres, the meshes of this knitted fabric being sufficiently wide to allow the passage of air while, at the same time, being sufficiently narrow to divide or calibrate the drops of rainwater.

2. A covering according to claim 1, characterized in that the *knitted* fabric (5) is made of glass fibre threads.

3. A covering according to any one of claims 1 or 2, characterized in that the *knitted* fabric (5) is coated by a resin having an excellent resistance to aging under the action of water and ultra violet radiation, this resin not completely closing the interstices between the threads of mineral fibres.

4. A covering according to any one of claims 1 to 3, characterized in that the meshes of the knitted fabric (5) are sufficiently narrow to prevent the passage of water, while permitting the passage of air.

5. A covering according to claim 4, characterized in that it is constituted by several layers (9, 10, 11) of *knitted* fabric (5).

6. A covering according to claim 5, characterized in that it is constituted by several layers (9, 10, 11) of *knitted* fabric (5) separated by laths (12).

7. Use of a covering according to one of any of the claims 1 to 6 for the heat insulation and protection against the wind of dwelling houses (1), characterized in that the roof (3) and the walls (2) of these houses (1) are covered by the said covering made of *knitted* fabric (5) and leaving an empty space (6) between the *knitted* fabric (5), the roof (3) and the walls (2).

## Ansprüche

1. Aus Mineralfasern hergestellte geschmeidige Verkleidung für die Wärmeisolation und für den Schutz eines Gebäudes oder eines starren Bauwerks gegen Witterungseinflüsse wie Wind und Regen, wobei die Verkleidung die Außenfläche des Gebäudes (1) oder des Bauwerks (15) bedeckt, dadurch gekennzeichnet, daß sie aus einem Wirkstoff (5) aus Mineralfasern besteht, dessen Maschen locker genug sind, um Luft hindurch zu lassen, jedoch eng genug sind, um Regentropfen zu zerteilen oder gleich groß zu machen.

2. Verkleidung nach Anspruch 1, dadurch gekennzeichnet, daß der Wirkstoff (5) aus Glasfaserfäden besteht.

3. Verkleidung nach Anspruch 1 oder 2, dadurch gekennzeichnet, daß der Wirkstoff (5) mit einem Harz mit ausgezeichneter Beständigkeit gegen Alterung durch Wasser und durch Ultraviolettstrahlung bestrichen ist, wobei dieses Harz die Zwischenräume der Mineralfaserfäden nicht vollständig verschließt.

4. Verkleidung nach einem der Ansprüche 1 bis 3, dadurch gekennzeichnet, daß die Maschen des Wirkstoffs (5) genügend eng sind, um den Durchtritt von Wasser zu verhindern, jedoch den Luftdurchtritt zu gestatten.

5. Verkleidung nach Anspruch 4, dadurch gekennzeichnet, daß sie aus mehreren Schichten (9, 10, 11) des Wirkstoffs (5) besteht.

6. Verkleidung nach Anspruch 5, dadurch gekennzeichnet, daß sie aus mehreren Schichten (9, 10, 11) des Wirkstoffs (5) besteht, die durch Distanzleisten (12) voneinander getrennt sind.

7. Verwendung der Verkleidung nach einem der Ansprüche 1 bis 6 für die Wärmeisolation und für den Windschutz von Wohngebäuden (1), dadurch gekennzeichnet, daß das Dach (3) und die Mauern (2) des Wohngebäudes (1) mit der durch den Wirkstoff (5) gebildeten Verkleidung bedeckt werden, wobei zwischen dem Wirkstoff (5), dem Dach (3) und den Mauern (2) ein Raum (6) freigelassen wird.

FIG_1

FIG_2

FIG_5

# FIG_3

# FIG_4

# FIG_6